# EUROPEAN PATENT SPECIFICATION

(11) **EP 2 968 157 B1**
(45) Date of publication and mention of the grant of the patent: **08.11.2023**
(21) Application number: 14763372.1
(22) Date of filing: 14.03.2014
(51) Int. Cl.: A61K 9/14, A61Q 19/00, A61Q 19/08, A61K 8/33, A61K 8/24, A61K 31/375, A61K 31/385, A61P 17/00, A61K 9/00, A61K 45/06, A61K 47/02, A61K 31/05, A61K 31/11, A61K 31/355

(54) **FINE DRY PARTICULATE RETINOID ACTIVE AGENT COMPOSITIONS AND TOPICAL FORMULATIONS INCLUDING THE SAME**
FEINE, TROCKENE, TEILCHENFÖRMIGE WIRKSTOFFZUSAMMENSETZUNGEN FÜR DIE NETZHAUT UND TOPISCHE FORMULIERUNGEN DAMIT
COMPOSITIONS RÉTINOÏDES PARTICULAIRES FINES ET SÈCHES ET FORMULATIONS TOPIQUES LES COMPRENANT

(30) Priority: 15.03.2013 US 201361798758 P; 10.02.2014 US 201461938080 P
(43) Date of publication of application: 20.01.2016
(73) Proprietor: Laboratory Skin Care, Inc., Tahoe City, California 96145 (US)
(72) Inventor: CHEN, Xin, Menlo Park, California 94025 (US); CANELIDE, Rodica-Tatiana, Menlo Park, California 94025 (US); MANSOURI, Zahra, Tahoe City, California 96145 (US)
(74) Representative: Stevens Hewlett & Perkins
(86) International application number: PCT/US2014/029673
(87) International publication number: WO 2014/145034

(56) References cited:
- WO-A1-2010/050980
- US-A- 6 096 324
- US-A1- 2007 014 863
- US-A1- 2010 086 606
- US-A1- 2010 330 014
- US-A1- 2011 189 239
- US-A1- 2011 280 943
- YAPAR ET AL.: 'Nanomaterials and Cosmetics;' J. FAC. PHARM. ISTANBUL vol. 42, no. 1, 2012, pages 43 - 70, XP055292941
- MIHRANYAN ET AL.: 'CURRENT STATUS AND FUTURE PROSPECTS OF NANOTECHNOLOGY IN COSMETICS' PROGRESS IN MATERIALS SCIENCE vol. 57, no. 5, 2012, pages 875 - 910, XP055292942

## Description

The present invention relates to a shelf-life stable dry particulate retinal composition and to a topical formulation comprising such a composition.

### INTRODUCTION

Skin includes a surface layer, known as the epidermis, and a deeper connective tissue layer, known as the dermis. The epidermis undergoes continuous turnover as the outermost cells are exfoliated and replaced by cells that arise from inner dermal layers. The dermis is composed of a variety of cell types, including fibroblasts.

Skin thickness begins to decline in humans after the age of 20 as the dermis becomes thinner and the number of skin fibroblasts declines. As skin ages, or is exposed to UV light and other environmental insults, changes in the underlying dermis can lead to the functional and morphological changes associated with damaged skin. Decreases in the abundance and function of products of the fibroblasts, which include collagen and proteoglycans, are believed to play major roles in wrinkled and damaged skin.

Retinol and its derivatives display key regulatory functions in epidermal growth and differentiation. Topically-applied retinol reduces fine lines and wrinkles by skin absorption, which leads to increases in the rate of collagen production. However, retinol is sensitive to oxidation and photolysis, and rapidly converts to the cis-isomer and degrades when exposed to light or oxygen. Retinoinds, such as retinoic acid, are a group of biochemical agents proven to reduce fine lines, wrinkles and other signs of skin aging. Retinaldehyde is the direct precursor of retinoic acid in the metabolic process, combining the effectiveness retinoids with lower potential for skin irritation. Like other retinoids, retinaldehyde has stability issues and will degrade if it is exposed to sunlight or certain temperatures/humidity levels.

WO2010/050980 discloses ceramic calcium phosphate carrier (Hydroxysomes) with retinoic acid in a topical composition for topical application to the skin. US2010/086606 discloses Hydroxysomes with retinyl palmitate.

### SUMMARY

The invention is set out in the appended set of claims.

Fine dry particulate retinoid compositions suitable for use in topical formulations, as well as methods of making the same, are provided. In the dry particulate retinoid compositions, the retinoid active agent is associated with the particles, e.g., via entrapment in the pores of the particles and/or ionic binding and/or non-covalent binding to the surface of the particles and/or loosely associated with the particles. Also provided are topical formulations which include the dry particulate retinoid compositions of the invention, and methods of using the same.

### DETAILED DESCRIPTION

Fine dry particulate retinoid compositions suitable for use in topical formulations, as well as methods of making the same, are provided. In the dry particulate retinoid compositions, the retinoid active agent is associated with the particles, e.g., via entrapment in the pores of the particles and/or ionic binding and/or non-covalent binding to the surface of the particles and/or loosely associated with the particles. Also provided are topical formulations which include the dry particulate retinoid compositions of the invention, and methods of using the same.

Before the present invention is further described, it is to be understood that this invention is not limited to particular embodiments described, as such may vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Methods recited herein may be carried out in any order of the recited events which is logically possible, as well as the recited order of events.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

All publications mentioned herein describe the methods and/or materials in connection with which the publications are cited.

It must be noted that as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### METHODS OF MAKING FINE DRY PARTICULATE ACTIVES AND FINE DRY PARTICULATE ACTIVES PRODUCED USING THE SAME

As summarized above, aspects of the invention include methods of making fine dry particulate retinoid compositions, where the methods include combining an amount of nanoporous calcium particles (e.g., calcium phosphate particles) and one or more retinoid active agents in a manner sufficient to produce a dry particulate retinoid composition. As reviewed above, in the dry particulate retinoid compositions, the active agent is associated with the particles, e.g., via entrapment in the pores of the particles and/or ionic binding and/or non-covalent binding to the surface of the particles and/or loosely associated with the particles. In practicing methods according to embodiments of the invention, nanoporous calcium particles and one or more retinoid active agents are combined in the presence of a suitable solvent system under conditions sufficient for the active agent(s) to enter internal space of the particles and/or ionically bind and/or covalently bind and/or associate with the surface of the particles. Before further describing the method steps, the particles, active agents and solvent systems employed in certain embodiments of the methods are reviewed in greater detail.

### Nanoporous calcium particles

Particles employed in methods of the invention are nanoporous phosphate particles. By "nanoporous" is meant that the particles have a porosity of 30% or more, such as 40% or more, including 50% or more, where the porosity may range from 30% to 85%, such as from 40% to 70%, including from 45% to 55%, as determined using a mercury intrusion porosimeter porosity determination protocol as described in ASTM D 4284-88 "Standard Test Method for Determining Pore Volume Distribution of Catalysts by Mercury Intrusion Porosimetry". Porosity is also described by "pore volume (ml/g)" and in such instances many range from 0.1 ml/g to 2.0 ml/g. In some cases, the particles have a porosity such that their internal surface area ranges from 10 m²/g to 150 m²/g, such as from 20 m²/g to 100 m²/g, including 30 m²/g to 80 m²/g, as determined using a BET gas adsorption surface area determination protocol as described in ASTM D3663-03 Standard Test Method for Surface Area of Catalysts and Catalyst Carriers. The pore diameter may vary, ranging in certain instances from 2 to 100 nm, such as 5 to 80 nm, including 10 to 60 nm. In addition, the particles may have a tapping density ranging from 0.2 g/cm³ to 0.5 g/cm³, such as from 0.25 g/cm³ to 0.45 g/cm³, including from 0.3 g/cm³ to 0.4 g/cm³. The tap density can be measured by using standard ASTM WK13023 - New Determination of Tap Density of Metallic Powders by a Constant Volume Measuring Method.

In some instances, the particles are rigid particles which are uniform and spherical in shape. By "rigid" is meant that the particles are hard, such that they are not pliant. By "uniform" is meant that the shape of the particles does not vary substantially, such that the particles have substantially the same spherical shape. The term "spherical" is employed in its conventional sense to mean a round body whose surface is at all points substantially equidistant from the center. Of interest in certain embodiments are calcium particles having a diameter of 20 µm or less, such as 10 µm or less, including 5 µm or less, where in some instances the medium diameter is 4 µm or less, such as 3 µm or less, including 2 µm or less. Of interest in certain embodiments are calcium particulate compositions in which the median diameter of the all of the particle members in the composition is 20 µm or less, such as 10 µm or less, including 5 µm or less, where in some instances the medium diameter is 4 µm or less, such as 3 µm or less, including 2 µm or less. Of interest in certain embodiments are calcium particlate compositions in which the arithmetic mean or average of all of the particles in the composition is 20 µm or less, such as 10 µm or less, including 5 µm or less, where in some instances the medium diameter is 4 µm or less, such as 3 µm or less, including 2 µm or less. With respect to the above ranges, in some instances the particles have a diameter of 0.1 µm or greater, such as .05 µm or greater, including 1.0 µm or greater.

The particles are, in some instances, chemically pure. By chemically pure is meant that the particles are made up of substantially one type of compound, e.g., a calcium compound, such as a calcium phosphate mineral. Of interest as porous particles are calcium containing particles, such as calcium containing particles that are made of a molecule that includes calcium cation and a suitable anion, e.g., carbonate, phosphate, etc. In some instances, the particles are calcium carbonate particles, such as but not limited to the calcium carbonate particles disclosed in U.S Patent Nos. 5,292,495 and 7,754,176. In some instances, the calcium phosphate particles are made up of a calcium phosphate that is described by the molecular formula Ca₁₀(PO₄)₆(OH)₂.

In some instances, the particles are ceramic particles. By ceramic is meant that the particles are produced using a method which includes a step of subjecting the particles to high temperature conditions, where such conditions are illustrated below. High temperatures may range from 200 to 1000°C, such as 300 to 900°C and including 300 to 800°C. In some embodiments, the particles have a compression rupture strength ranging from 20 to 200 MPa, such as from 50 to 150 MPa, and including 75 to 90 MPa, as determined using a SHIMADZU MCT-W500 micro-compression testing machine particle strength determination protocol with a particle sintered at temperature of 400°C to 900°C, as described in European Patent EP1840661. In some embodiments, the particles are biodegradable, by which is meant that the particles degrade in some manner, e.g., dissolve, over time under physiological conditions. As the particles of these embodiments are biodegradeable under physiological conditions, they at least begin to dissolve at a detectable rate under conditions of pH of 5.8 or less, such as 5.5 or less, e.g., 5.3 or less, including 5 or less, e.g., 4.9 or less.

The uniform, rigid, spherical, nanoporous calcium phosphate particles employed in embodiments of the methods may be prepared using any convenient protocol. In one protocol of interest, the particles are manufactured by spray drying a slurry which includes nanoporous calcium phosphate (e.g., hydroxyapatite) crystals (which may range from 2nm to 100 nm size range) to produce uniform spherical nanoporous calcium phosphate particles. The resultant particles are then sintered for a period of time sufficient to provide mechanically and chemically stable rigid spheres. In this step, the sintering temperatures may range from 100 °C to 1000 °C, such as 200°C to 1000°C, such as 300°C to 900°C and including 300°C to 800°C for a period of time ranging from 1 hour to 10 hours, such as 2 hours to 8 hours and including 3 hours to 6 hours.

In some instances, the nanoporous calcium particles may be pre-treated. Pretreated particles may be prepared via a number of different protocols. In some instances, the particles may be neutralized with a pH adjuster, e.g., such as an acid. The pH may be adjusted to optimum range, which may be specific to the active agent, when necessary. Examples of pH adjusters of interest include weak or strong acids such as hydrochloric acid, glycolic acid, phosphoric acid, lactic acid and citric acid and others. In some instances, the particles may be pretreated with a phosphate salt, such as sodium phosphate or pretreated with a calcium salt, such as calcium chloride. In some instances, a mixture of buffering system is used such as sodium citrate and citric acid or calcium chloride and lactic acid. Where desired, any salts produced during this protocol may be removed, e.g., via filtering or decanting.

In a given amount of the fine dry particulate composition, the weight percent of the calcium particles may vary. In some instances, the weight percent ranges from 10 to 95 wt.%, such as 20 to 90 wt.%, e.g., 25 to 90 wt. %, 50-90 wt.%, 50 to 75 wt.%, including 55 to 65 wt.%.

### Retinoid Active Agent

The term "retinoid active agent" refers to an agent that has retinoid activity. A retinoid in the composition can include both naturally occurring and synthetic compounds having the general structure of vitamin A (retinol) and variations of that structure having similar biological and pharmacological activity as retinol. Examples of retinoids include, but are not limited to, all-trans retinol, retinol, retinal, retinyl acetate, retinaldehyde, retinyl palmitate, retinoic acid, retinyl propionate, retinyl linoleate, dehydroretinol, eretinate, eretrin, motretinide, a synthetic retinoid, and mixtures thereof. U.S. Pat. No. 5,851,538 discloses several additional useful retinoids.

In a given amount of the fine dry particulate composition, the weight percent of the retinoid active agent may vary. In some instances, the weight percent ranges from 0.1 to 50 wt.%, such as 1 to 30 wt.%, e.g., 2 to 25 wt. %, including 15 to 25 wt.%.

### Antioxidant

In some instances, the retinoid is stabilized by a single antioxidant or antioxidant mixture. In such instances, the fine dry particulate actives may be made by selecting a desired antioxidant or mixture of antioxidants and dissolving in a suitable organic- or water-based solvent. For organic-based solvents, retinaldehyde is added to the solution such that it too is dissolved. Once dissolved, calcium particles, e.g., as described above, are added to the solution and the suspension evaporated to dryness through roto-evaporation, spray drying or other suitable method. For water-based solvents, the antioxidants are dissolved in a water-based solution. Calcium particles, e.g., as described above, are then added to the solution and the solution evaporated to dryness through roto-evaporation, spray drying or other suitable method. Retinaldehyde is then dissolved in an organic-based solvent to which the dried antioxidant- alcium particles, e.g., as described above, are added. The resulting suspension is evaporated to dryness through roto-evaporation, spray drying or other suitable method.

Antioxidants can be a mixture of many different one o rmore antioxidants from the following list: Acacia Victoriae Fruit Extract, Acer Palmatum Leaf Extract, Acetamidocaproic Acid, Acetyl Benzoyloxy Prasterone, Acetyl Cysteine, 2-Acetylhydroquinone, Agrimonia Eupatoria Root Extract, Alchemilla Vulgaris Leaf Extract, Alpinia Uraiensis Stalk / Leaf Water, Aminoethanesulfinic Acid, Aminopropyl Andrographolide, Ascorbyl Phosphate, Aminopropyl Tocopheryl Phosphate, Anserine, Apigenin, Arabidopsis Thaliana Extract, Arbutin, Alpha- Arbutin, Arctium Lappa Fruit Extract, Asarum Heterotropoides Rhizome Extract, Ascorbic Acid, Ascorbic Acid Polypeptide, Ascorbyl Dipalmitate, Ascorbyl Glucoside, Ascorbyl Linoleate, Ascorbyl Methylsilanol Pectinate, Ascorbyl Palmitate, Ascorbyl Stearate, Ascorbyl Tetraisopalmitate, Ascorbyl Tocopheryl Maleate, Asiaticoside, Avena Sativa (Oat) Kernel Extract, Astaxanthin, Bacillus/Rice Bran Extract/ Soybean Extract Ferment Filtrate, Benzoguanamine, Beta Vulgaris (Beet) Root Extract, BHA, BHT, Bis-demethoxycurcumin, Buddleja Axillaris Leaf Extract, Butylated Xylenol, 4-Butylresorcinol, Caffeic Acid, Calcium Ascorbate, Calophyllum Inophyllum Seed Oil, Camellia Sinensis Cathechins, Camellia Sinensis Leaf Extract, Camellia Sinensis Leaf Oil, Capparis Moonii Fruit Extract, Carnosic Acid, Carotenoids, Cayaponia Tayuya Root Extract, Cercis Chinensis Flower / Leaf/ Stem Extract, Chitosan Ascorbate, Chitosan Glycolate, Chitosan Salicylate, Chlorogenic Acids, Chrysanthemum Boreale Flower Extract, Cimicifuga Dahurica Root Extract, Citrus Junos Seed Extract, Cobalt DNA, Copper Adenosine Triphosphate, Coptis Chinensis Root Extract, Crotonaldehyde, Curcumin, Cyamopsis Tetragonoloba (Guar) Symbiosome Extract, Cyclopia Genistoides Leaf Extract, Cysteine, Cysteine HCI, Davidsonia Pruriens Fruit Extract, Decyl Mercaptomethylimidazole, Demethoxycurcumin, Diacetylcurcumin, Diamylhydroquinone, Di-T - Butylhydroquinone, Dicetyl Thiodipropionate, Digalloyl Trioleate, Dilauryl Thiodipropionate, Dimethoxy Di-p-Cresol, Dioleyl Tocopheryl Methylsilanol, Diosmine, Disodium Ascorbyl Sulfate, Disodium Rutinyl Disulfate, Disodium Salicylphosphate, Disodium Ubiquinone, Distearyl Thiodipropionate, Dodecyl Gallate, Dunaliella Bardawil Powder, Ellagic Acid, Epigallocatechin Gallate, Epimedium Sagittatum Leaf / Stem Extract, Ergothioneine, Eriobotrya Japonica Leaf Protoplast, Erythorbic Acid, Ethyl Ferulate, Ethylhexyl Ferulate, Ethylhexyl Gallate, Euterpe Oleracea (Acai berry) Extract, Ferulic Acid, Foeniculum Vulgare (Fennel) Seed Extract, Fragilaria Pinnata Extract, Furfuryl Palmitate, Genistein Glucoside, Ginkgo Leaf Terpenoids, Glucosylrutin, Glutathione, Glycine Soja (Soybeen) Oil, Glycyrrhiza Glabra (Licorice) Root Extract, Glycyrrhiza Glabra (Licorice) Root Water, Grifola Frondosa (Maitake) Mycelium Ferment Filtrate Extract, Haematococcus Pluvialis Extract, Haematococcus Pluvialis Oil, Haematococcus Pluvialis Powder, Hesperetin, Hesperetin Laurate, Hesperidin Methyl Chalcone, Honokiol, Hydrolyzed Aspergillus / Ginseng Extract Ferment, Hydrolyzed Gardenia Florida Extract, Hydrolyzed Proanthocyanidin, Hydrolyzed Soy Extract, Hydroquinone, p-Hydroxyanisole, Hydroxydecyl Ubiquinone, Hydroxylamine HCI, Hydroxylamine Sulfate, Hypericin, Inositol Hexaniacinate Hexaascorbte, Isooctyl Thioglycolate, Isoquercetin, Kaempferol, Kojic Acid, Kojyl Glucoside, Kojyl Methylenedioxycinnamate, Lens Culinaris (Lentil) Symbiosome Extract, Ligusticum Striatum Root Extract, Linseed Oil Ascorbate Esters, Lotus Japonicus Symbiosome Extract, Lutein, Lycopene, Madecassoside, Magnesium Ascorbate, Magnesium Ascorbate/ PCA, Magnesium Ascorbyl Phosphate, Magnolol, Malpighia Punicifolia (Acerola) Fruit Extract, Manganesse Adenosine Triphosphate, Manganesse Dioxide, Mangiferin (Mangifera Indica), Mangosteen (Garcinia Mangostana), Medicago Sativa (Alfalfa) Symbiosome Extract, Melaleuca Alternifolia (Tea Tree) Leaf Oil, Melatonin, Methoxy PEG-7 Ascorbic Acid, Methoxy PEG-7 Rutinyl Succinate, Methyl Di-t-butyl Hydroxyhydrocinnamate, Methylsilanol Ascorbate, Monascus/ Rice Ferment, Niacinamide Hydroxybenzoate, Nordihydroguaiaretic Acid, Octanicotinoyl Epigallocathechin Gallate, Olea Europaea (Olive) Fruit Unsaponifiables, Palmitoyl Camellia Sinensis Extract, Palmitoyl Grape Seed Extract, Phenylthioglycolic Acid, Phloroglucinol, Pikea Robusta Extract, Piper Negrum (Pepper) Seed, Pisum Sativum Symbiosome Extract, Pomegranate (Punica Granatum),Potassium Ascorbyl Tocopheryl Phosphate, Potassium Sulfite, Propyl Gallate, Pyridoxine Hydroxybenzoate, Pyridoxine Hydroxycitrate, Quercetin, Resveratrol, Retinyl Formyl Aspartame, Rosmarinic Acid, Rosmarinus Officinalis (Rosemary) Flower Extract, Rosmarinus Officinalis (Rosemary) Leaf Extract, Rutin, Rutin hydrate, Sodium Ascorbate, Sodium Ascorbyl/ Cholesteryl Phosphate, Sodium Ascorbyl Phosphate, Sodium Bisulfite, Sodium Erythorbate, Sodium Metabisulfite, SOD, Superoxide Oxidase, Sodium Sulfite, Sodium Tocopheryl Phosphate, Stearyl Gallate, Tetrahexyldecyl Ascorbate, Tetrahydrocurcumin , Tetrahydrocurcumin Diacetate, Thioctic Acid (Alpha Lipoic Acid), Thiodiglycolamide, Thiodiglycolic acid, Thiolactic Acid, Thiotaurine, Tocopherol, Tocopheryl Acetate, Tocopheryl Linoleate, Tocopheryl Nicotinate, Tocopheryl Retinoate, Tocopheryl Succinate, Tocoquinone, Trisodium Ascorbyl Isopalmitate Phosphate, Ubiquinol, Ubiquinone (Co Q-10), Vitis Vinifera (Grape) Juice Extract, Vitis Vinifera (Grape) Seed Extract, Vitis Vinifera (Grape) Seed Extract, Zinc Adenosine Triphosphate, Zinc Ascorbate.

As reviewed above, a given antioxidant component may be made up of a single antioxidant or two more different antioxidants, e.g., three or more, four or more, five or more antioxidants. In some instances, the antioxidant component includes two antioxidants.

Where the antixodiant component includes two or more antioxidants, any convenient combination may be employed. For example, BHT plus Ascorbyl Palmitate, Thioctic Acid (Alpha Lipoic Acid) plus Calcium Ascorbate, Tocopherols (e.g., mixed tocopherols) plus Thioctic Acid (Alpha Lipoic Acid) plus Resveratrol, Tocopherols plus Thioctic Acid (Alpha Lipoic Acid), etc. Where the antioxidant component includes two or more antioxidants, the weight ratio of the antioxidants may vary, where the weight ratio may be equal, e.g., 1:1, or not equal, e.g., ranging from 0.1:5 to 5:0.1, such as 1:2.5 to 2.5:1.

In a given amount of the fine dry particulate composition, the weight percent of total antioxidant componet (made up of one or more antioxidants, such as described above) may vary. In some instances, the weight percent ranges from 1 to 50 wt.%, such as 5 to 30 wt.%, e.g., 5 to 25 wt. %, 5 to 20 wt.%, 15-20 wt.%.

It is noted that the above describe antioxidant compositions are not limited to use with retinoid active agents, e.g., as described herein. Instead the antioxidant systems described herein find use with a variety of different active agents when complexed with calcium particles, including but not limited to those compositions described in U.S. Patent No. 8,445,002.

### Solvent System

The solvent system may be made up of a single solvent or two or more different solvents, where the particular solvent or solvents making up the solvent system may be selected based on the nature of active agent to be complexed with the particles. In some instances, the solvent system is aqueous, and may be 100% water, or water in combination with one or more additional solvents, including polar and non-polar solvents, which may be organic or inorganic, as desired. In other instances, the solvent system may be non-polar.

### Fabrication of Dry Particulate Actives

As summarized above, in preparing dry particulate actives in accordance with embodiments of the invention, the active agent(s), nanoporous calcium phosphate particles and solvent system are combined to produce a calcium phosphate particles/active agents mixture. The various components may be combined using any convenient protocol. In some instances, the active agent(s) is first dissolved in the solvent system, and then the resultant active agent solution is combined with an amount of calcium phosphate particles. In yet other instances, the calcium phosphate particles are combined first with the solvent system, and then the active agent is added to produce the calcium phosphate particles/active agents mixture.

The active agent(s) and solvent system may be combined using any protocol sufficient to produce the desired mixture solution. In some instances, the active agent(s) and solvent system are combined with agitation. Agitation may be provided using any convenient protocol, e.g., stir bar, agitation blade, propeller, etc. The temperature at which the active is combined with the solvent system and dissolved therein may vary, and may be below room temperature, at room temperature or above room temperature. The specific temperature at which the combination of active agent and solvent is carried out may be chosen based on the nature of the active agent (such that a temperature is chosen that will not inactivate the active agent) as well as the properties of the solvent system, e.g., melting point, boiling point, etc. In some instances, the temperature ranges from just above 0°C to 200°C. In some instances, the temperature ranges from 4 to 25°C, e.g., 5 to 10 °C. In some instances, the temperature is above room temperature, e.g., 35 to 60°C, e.g. 40 to 45 °C, 50 to 55°C, or higher. In some instances, the temperature ranges from 65 to 150°C, e.g. 70 to 85 °C, 90 to 105°C, 120 to 135°C or higher. In some instances, the temperature ranges from 5 to 80°C, such as 5 to 75°C, e.g., 10 to 65 °C, e.g., 20 to 60 °C.

The amount of active agent that is dissolved in the solvent system may be selected based on the solubility of the active agent in the solvent system and/or based on the amount of calcium phosphate particles to be used. In some instances, the amount of active agent relative to the calcium phosphate particles is 0.1% by weight or more, such as 10% by weight or more, such as 20% by weight or more, such as 30% by weight or more, such as 40% by weight or more, such as 60% by weight or more, such as 70% by weight or more, such as 80% by or more, such as 90% by weight or more, including 100% by weight or more, including 1000% by weight or more. In some instances, the weight ratio of active agent(s) to calcium phosphate particles ranges from 0.01:10, 0.1:1, 1:1 and 1:0.1. In some instances, the weight ratio of active agent(s) to calcium particles ranges from 0.5:1.0 to 5:1, where in some instances the ratio is 1:1.

Following preparation of the active agent solution, e.g., as described above, a suitable amount of calcium phosphate particles (which may or may not be pre-treated, e.g., as described and referenced above) is combined with the solution. In some instances, the calcium phosphate particles that are combined with the active agent solution are dry. In some instances, the methods include wetting an initial amount of nanoporous calcium phosphate particles with a solvent system, where the solvent system may be the same as or different from that used to prepare the active agent solution, e.g., as described above.

The particles (either dry or wetted as described above) may be combined with a solution of an active agent present in a solvent system, e.g., as described above, to produce a liquid composition that includes particles and an active agent(s) in a solvent system, which composition may be referred to herein as an active agent mixture. The active agent solution and particles (dry or wetted, as desired) may be mixed using any convenient protocol, e.g., with agitation (such as described above), to produce a liquid composition that includes both the particles and the active agent in a solvent system. This mixing step lasts for a time sufficient to produce the desired mixture, and in some instances ranges in length from 1 minute to 600 minutes, such as 5 minutes to 300 minutes. In certain instances, the nanoporous calcium phosphate particles and active agent(s) solution are combined under negative pressure. When combined under negative pressure, pressures of interest may vary and in some instances range from 0.001 torr to 1 torr, such as 0.01 torr to 0.1 torr and including 0.05 torr to 0.5 torr.

Following preparation of the mixture, the solvent system is dried off from the active agent mixture to produce the desired fine dry particulate active. Drying may be accomplished using any convenient protocol, where protocols of interest include, but are not limited to: maintaining at elevated temperatures sufficient to evaporate the solvent. Drying methods of interest include, but are not limited to: drying by heat convection, such as spray drying, air flow drying, fluid bed drying, and super-heated steam drying, or drying by heat conduction, such as vacuum drying, freeze drying, rotary drum drying, and rotary vacuum drying or drying by heat radiation, such as infrared heat drying and microwave drying, or heat radiation with other electromagnetic waves, and or other methods such as super critical drying, etc. Combinations of various protocols may be employed, as desired. Following separation of the solvent, the resultant dry product may be further processed as desired, e.g., the product may be grinded, milled (e.g., via ball mill, hammer mill, jet impact mill, wet impact mill, etc.), sieved (e.g., with or without vibration, subjected to air-flow or jet-flow classification), etc., as desired, to produce a fine dry particulate active.

As indicated above, the active compositions of the invention may be characterized by having a single active agent associated with given calcium particles, or two or more active agents (e.g., three or more active agents, four or more, five or more) different active agents associated with the same calcium particles.

The above fabrication protocol results in the production of a fine dry particulate retinoid active of the invention. In the resultant dry powder active agent is present inside of the particles, and/or bound to the particles, covalently or ionically, and/or on the surface of the particles, and/or tightly associated with the particles and loosely associated with the particles. The amount of active agent component (which is made up of one or more distinct active agents) that is bound or associated with calcium phosphate particles may vary depending on the particular active agent(s). The resultant active particulate has a distribution of diameter of the particles, where in some instances the majority (such as 60% or more, 75% or more, 90% or more, 95% or more) of the particles have diameters that range from 0.01 to 100 µm, such as from 0.01 to 20 µm, such as from 0.1 to 10 µm, and including from 0.1 to 2 µm.

In some instances, the amount of active agent relative to the calcium particles ranges from 1% or less by weight to 500% by weight or more, e.g., in some instance being 50% by weight or more, such as 60% by weight or more, such as 70% by weight or more, such as 80% by or more, such as 90% by weight or more, including 100% by weight or more, such as 150% by weight or more, e.g., 500% by weight or more, including 1000% by weight or more. In some instances, the weight ratio of active agent(s) to calcium particles ranges from 0.5:1.0 to 5:1, e.g., 0.1 to 1 to 1:0.1, where in some instances the ratio is 1:1.

Depending on the nature of the resultant active to be employed, the protocols may or may not include a step of coating the resultant active powder. Coating materials (which may include one or more coating material) of interest are those that preserve the association of the active agent with the calcium phosphate particles in various formulations, e.g. formultions designed for topical application to the skin. Suitable coating agents include agents that are physiologically acceptable and are solid at room temperature and are suitable for application to the skin. The coating material component may be a single material or a combination of two or more materials, e.g., where the combination provides for one or more desirable properties. Materials that find use as coating materials include, but are not limited to waxes, butters, etc. Coatings materials of interest and methods for their use are further described in U.S. Patent Application No. 12/565,687 published as US 2010-0086606 A1.

### TOPICAL FORMULATIONS

Aspects of the invention further include topical formulations that are configured for application to a topical site of a human subject. Topical formulations of the invention are for applications such as mucosal surface or keratinized skin surface of a mammalian subject, such as a human subject. By mucosal surface is meant a location of a subject that includes a mucosal membrane, such as the inside of the mouth, in the inside of the nose, etc. By keratinized skin surface is meant a skin location of a subject, i.e., a location of the external covering or integument of an animal body. Because the topical formulations of the invention are formulated for delivery to topical location, they are formulated so as to be physiologically compatible with the topical location for which they are formulated. Accordingly, when contacted with the target keratinized skin surface for which they are formulated, the topical compositions do not cause substantial, if any, physiological responses (such as inflammation or irritation) that would render the use of the topical compositions unsuitable for topical application. Topical formulations of the invention include: (a) an amount of the actives (which may or may not be stabilized); and (b) a topical delivery vehicle.

As indicated above, the topical compositions include an amount of the fine dry particulate active present in a topical delivery vehicle. The amount of fine dry particulate active that is present in the delivery composition and therefore combined with a delivery vehicle may vary. In some embodiments, the amount of fine dry particulate active present in the delivery vehicle ranges from 0.01mg/g to 500 mg/g, such as 0.01 to 250 mg/g, such as 0.1 to 200 mg/g, e.g., 1 to 100 mg/g, including 10 to 50 mg/g fine dry particulate active per gram of delivery vehicle. In certain embodiments the fine dry particulate active are present in compositions in an amount ranging from about 0.001% or more by weight, such as 0.01%, or 0.05%, or 1% or more, 5% or more, 10% or more, 15% or more, 25 % or more, 30% or more 50% or more. In certain embodiments, the fine dry particulate active is added directly to the delivery vehicle (i.e., the fine dry particulate active is not wetted prior to combining/mixing with the delivery vehicle). In other words, the fine dry particulate active and the delivery vehicle are combined to form the topical composition.

The delivery vehicle (i.e., topical delivery component) refers to that portion of the topical composition that is not the fine dry particulate active. Delivery vehicles of interest include, but are not limited to, compositions that are suitable for applications via one or more of oral, topical, implantation, ocular, aural, rectal, vaginal, etc., routes. In certain embodiments, the vehicle is formulated for application to a topical region or surface of a subject, such as a keratinized skin surface. The subject compositions may be formulated as stable solutions or suspensions of the components, e.g., in an aqueous solvent. Where desired, the components may be combined with one or more carrier materials to form a solution, suspension, gel, lotion, cream, ointment, aerosol spray, roll-on, foam products, mousses, powders, sticks, or the like, as desired. Of interest in certain embodiments are aqueous delivery vehicles, i.e. aqueous vehicles that include a certain amount of water. Examples of aqueous vehicles include hydrogel vehicles, sprays, serums, etc.

The topical composition may also contain other physiologically acceptable excipients or other minor additives, particularly associated with organoleptic properties, such as fragrances, dyes, buffers, cooling agents (e.g. menthol), coating materials or the like. The excipients and minor additives will be present in conventional amounts, e.g., ranging from about 0.001% to 5%, such as 0.001-2%, by weight, and in some instances not exceeding a total of 10% by weight.

Lotions (as well as other topical formulations) of interest may include one or more of the following components: Water, Viscosity modifiers, Humectants, Vegetable oils and hydrogenated vegetable oils, Emollients, Conditioning Agents, Emulsifiers, Glyceryl Esters of Fatty Acids, Silicone, C1-C30 monoesters and polyesters of sugar, Conditioning Agents, Preservatives, etc. Depending on the topical formulation, additional components of interest include: Abrasives, Absorbents, Antimicrobial and antifungal agents, Astringents, Anti-Acne agents, Anti-wrinkle agents, Anti-oxidants, Antimicrobials, Binders, Biological actives, Buffering actives, Bulking actives, Chelating agents, Chemical additives, External analgesics, Film former agents, Opacifying agents, pH adjusters, Reducing agents, Colorants, Fragrances, Cosmetic Soothing Agents, Tanning actives & accelerators, Skin lightening/whitening agents, Sunscreens, Surfactants, Skin Conditioning Agents, Vitamins, etc.

As indicated above, of interest in certain embodiments are semi-solid delivery compositions, such as gels, creams and ointments. Such compositions may be mixtures of (in addition to the active agent) water, water soluble polymers, preservatives, alcohols, polyvalent alcohols, emulsifying agents, wax, solvents, thickeners, plasticizers, pH regulators, water-retaining agents and the like. Furthermore, such compositions may also contain other physiologically acceptable excipients or other minor additives, such as fragrances, dyes, buffers, coating materials or the like.

Also of interest are solid formulations, such as topical patch formulations. Topical patch formulations may vary significantly. Topical patch formulations may include an active agent layer, a support and a release liner. The active agent layer may include physiologically acceptable excipients or other minor additives, such as fragrances, dyes, buffers, coating materials or the like. The support may be made of a flexible material which is capable of fitting in the movement of human body and includes, for example, plastic films, various non-woven fabrics, woven fabrics, spandex, and the like. Various inert coverings may be employed, which include the various materials which may find use in plasters, described below. Alternatively, non-woven or woven coverings may be employed, particularly elastomeric coverings, which allow for heat and vapor transport. These coverings allow for cooling of the pain site, which provides for greater comfort, while protecting the gel from mechanical removal. The release liner may be made of any convenient material, where representative release films include polyesters, such as PET or PP, and the like.

When present in the delivery vehicle, a high weight percentage of the active agent of the initial fine dry particulate composition may remain associated with the calcium particles. In some instances, the weight percentage that remains associated with the calcium particles (and therefore is not free in the delivery vehicle) is 40% or more, such as 50% or more, including 60% or more, e.g., 70% or more. Active agent that remains associated with the calcium particles may be carried along with the particles into the skin for delivery in the acidic environment of the skin.

### UTILITY

Topical formulations of the invention find use in methods of delivering active agents to a topical location of a subject, where the topical location may be a skin surface location or a mucosal location. In delivering active agents to a topical location of a subject, formulations of the invention may deliver the active agent at least into an epidermal location that is beneath the skin surface of a subject. As such, embodiments of the invention include methods of delivering active agent/calcium particle complexes into the stratum corneum of a subject, where the methods may result in delivery of the complexes into the deep stratum corneum and/or dermis of a subject. By "deep stratum corneum" is meant a region that is 1 or more cell layers below the skin surface, such as 2 or more, e.g., 5 or more cell layers below the skin surface, including 10 or more cell layers below the skin surface. In some instances, the active agent/calcium particle complexes are delivered to region of the stratum corneum that is 2 µm or more such as 5 µm or more and including 15 µm or more below the surface of the skin.

Upon reaching their target dermal location, the active agent/calcium particle complexes may begin to release their active agent "payload" and break down (e.g., via dissolution caused by pH gradient of the skin), as the uniform, rigid, spherical, nanoporous particles dissolve under acidic conditions, e.g., conditions of pH 5.5 or lower, such as 5 or lower, including 4.0 or lower, such as the physiological acidic conditions of the stratum corneum. The time required for dissolution of particles in the stratum corneum may vary, and in certain embodiments ranges from a few minutes up to several days, such as 1 minute to 24 hours, such as 10 minutes to 12 hours and including 30 minutes to 3 hours, over which time period active agent is released from the fine particulate dry active. The proportion of active agent that is released from the active agent/calcium particle complexes may vary, and in certain instances is 0.01 % or more, such as 0.1 % or more, including 1 % or more, such as 10 % or more, including 50 % or more, 75% or more, including up to 100% (w/w).

Methods of the invention may therefore result in delivery of an active agent at least into the stratum corneum of a subject. Additional target locations of the body of interest include additional epidermal regions, such as but not limited to the stratum lucidum, stratum granulosum, stratum spinusom, stratum basale and dermis. In certain embodiments, the active agent is delivered to a region of the dermis. In certain embodiments, the active agent is delivered to a region below the dermis, e.g., into subcutaneous tissues.

In practicing methods of the invention, a topical formulation is applied to a topical region of a subject and maintained at the topical region for a period of time sufficient to result in the desired delivery of active agent to the subject, as described above. The topical region is, in certain embodiments, a keratinized skin region. The keratinized skin region, including hair follicles, sweat glands and sebaceous glands, may be present at a variety of locations, e.g., limbs, arms, legs; torso, e.g., chest, back, stomach; head, e.g., neck, face; etc. In certain embodiments, the region will be a head region, such as a facial region, e.g., forehead, occipital region, around the mouth, etc. The topical region to which the composition is applied may vary with respect to area, ranging in certain embodiments from 1 mm² to 20,000 cm² or more, such as from 1 to 50 cm², and including from 3 to 10 cm².

Following application, the topical formulation is maintained at the site of application for a period of time sufficient for a desired therapeutic outcome to occur, e.g., amelioration of a symptom(s) of interest, reducing dryness. The period of time may vary, and in certain embodiments ranges from instantaneously up to several days, such as 1 min to 24 hours or longer, such as from 30 min to 12 hours and including from 1 hour to 12 hours or longer.

In practicing the methods of the invention, a subject may be administered a single dose or two or more doses over a given period of time. For example, over a given treatment period of one month, 1 or more doses, such as 2 or more doses, 3 or more doses, 4 or more doses, 5 or more doses, etc., may be administered to the subject, where the doses may be administered weekly or daily or even multiple times per day, with a holiday period in between, e.g., where the holiday period may vary, e.g., 4 hours, 6 hours, 12 hours, 1 day, 3 days, 7 days, etc.

The subject methods and compositions may be used in a variety of different kinds of animals, where the animals are typically "mammals" or "mammalian," where these terms are used broadly to describe organisms which are within the class mammalia, including the orders carnivore (e.g., dogs and cats), rodentia (e.g., mice, guinea pigs, and rats), lagomorpha (e.g., rabbits) and primates (e.g., humans, chimpanzees, and monkeys). In certain embodiments, the subjects or patients are humans.

The subject topical formulations find use in applications where it is desired to deliver a retinoid active agent to a subject. In certain embodiments, the subject topical formulations are employed in the treatment of a skin condition. By "treatment" is meant that at least an amelioration of the symptoms associated with the condition afflicting the subject is achieved, where amelioration is used in a broad sense to refer to at least a reduction in the magnitude of a parameter, e.g. symptom, associated with the condition being treated. As such, treatment also includes situations where the condition, or at least symptoms associated therewith, are completely inhibited, e.g. prevented from happening, or stopped, e.g. terminated, such that the subject no longer suffers from the condition, or at least the symptoms that characterize the condition. In certain embodiments a subject may be diagnosed for the presence of the disease condition, such that the topical formulations are provided to a subject known to be suffering from the disease condition.

Practice of methods of the invention can enhance result in the improvement in skin, when there is a noticeable decrease in the amount of wrinkling, roughness, dryness, laxity, sallowness, or pigmentary mottling of the treated skin. Methods of measuring improvements in skin condition are well known in the art (see, e.g., Olsen et al., J. Amer. Acad. Dermatol. 26:215-24, 1992), and can include subjective evaluations by the patient or a second party, e.g., a treating physician. Objective methods can include skin topography measurements, such as those described in Grove et al., J. Amer. Acad. Dermatol. 21 :631-37 (1989). In skin topography measurements, silicone rubber replicas are made of a small area of skin, e.g., a 1 cm diameter circular area. The silicone rubber replicas capture fine lines and wrinkles on the skin. These specimens are then analyzed using computerized digital image processing to provide an objective measurement of the skin's topography. Skin topography measurements generated following digital-image processing can be measured using the values Rₐ and R_{z} as described in Olsen et al., J. Amer. Acad. Dermatol. 37:217-26, 1997, where Rₐ represents the area of deviation of skin surface features above and below an average central line, and R_{z} represents the difference between the maximum and minimum heights in five equal segments of the skin surface profile. A statistically significant decline (e.g., P<0.05) in Rₐ and R_{z} values in skin treated according to the presence invention compared to untreated skin indicates an improvement in skin, as is achieved by practicing the methods of the invention.

Use of the compositions and methods of the invention provides for a number of important advantages. In some instances, the topical formulations (e.g., creams and lotions) that include the fine dry particulate retinoid active agent compositions are storage stable, such that the composition and/or active agent properties, e.g., color, viscosity, active gent activity, etc., are not substantially altered over extended periods of time, e.g., 1 week or longer, 2 weeks or longer, 1 month or longer, 6 months or longer, 1 year or longer, under room and elevated temperatures, e.g., 40°C or greater, including 50°C or greater. In some instances, the topical formulations (e.g., creams and lotions) that include the fine dry particulate retinoid active agent compositions exhibit increased bioavailability of the active agent as compared to a control, where the magnitude of increase may be 2 fold or greater, such as 5 fold or greater, including 10 fold or greater. In some instances, the topical formulations (e.g., creams and lotions) that include the fine dry particulate retinoid active agent compositions exhibit sustained releast of the active agent, where by sustained releast is meant release of therapeutically desired amount for 6 hours or longer, such as 12 hours or longer, including 18 hours or longer, e.g., 24 hours or longer, including 2 days or longer, e.g., 3 days or longer, 4 days or longer, 5 days or longer, 6 days or longer, 7 days or longer. In some instances, the topical formulations (e.g., creams and lotions) that include the fine dry particulate retinoid active agent compositions exhibit synergist results with respect to the skin health activitie of retinoid and calcium, where the magnitude of therapeutic results is greater than the expected additive activity of these two agents individually.

The following examples are offered by way of illustration and not by way of limitation.

### EXPERIMENTAL

### I. Preparation of Retinal-Hydroxysomes^{®} Calcium Phosphate Particulate Composition

a. A retinal- Hydroxysomes^{®} calcium chosphate particulate composition is prepared from 50-80% by weight Hydroxysomes^{®} calcium phosphate particles (Laboratory Skin Care, South San Francisco) and 10 to 30% by weight retinal powder. The dry components are combined by dissolving retinal in 200 to 600ml of ethanol until a clear solution is acheived, and then adding the Hydroxysomes^{®} calcium phosphate particles. The mixture is mixed at room temperature for 30 minutes, and then dried until no ethanol remains.
b. A retinal- Hydroxysomes^{®} calcium chosphate particulate composition is prepared from 50-80% by weight Hydroxysomes^{®} calcium phosphate particles (Laboratory Skin Care, South San Francisco), 10 to 30% by weight retinal powder and 5 to 50% by weight, including 5 to 20% by weight, of an antioxidant component made up of one or more antioxidants. The dry components are combined by first dissolving the antioxidant component in 200 to 600ml of ethanol until a clear solution is acheived, followed by dissolution of the retinal into the ethanol until a clear solution is acheived, and then adding the Hydroxysomes^{®} calcium phosphate particles. The mixture is mixed at room temperature for 30 minutes, and then dried until no ethanol remains. Antioxidant components employed include one or more of the following antixodants, such as two or more of the following antioxidants, including 3 or more of the following antioxidants: tocopherol, mixed tocopherols (e.g., a combination of 2 or more tocopherols, such as δ-tocopherol, γ-tocopherol, α-tocopherol, β-tocopherol), alpha lipoic acid, butylated hydroxytoluene, and ascorbyl palmitate.

### II. Characterization of Retinal-Hydroxysomes^{®} Calcium Phosphate Particulate Compositions

a. Compositions produced as described in I.a and I.b above are tested for shelf life stability. The products are observed to be stable at room temperature for 24 months, 40°C for 3 months and 50°C for 1 month as determined using an HPLC protocol.
   In addition, several formulations of the compositions are tested in water based topical formulations. Using HPLC analysis, the product was observed to be stable in the formulation at 50°C for one month, where the amounto of active agent decreased by less than 2.5%. Furthermore, several formulations of the compositions with an antioxidant component are tested in water based topical formulations. The results demonstrated that formulations having complexes with antixodant components as described above exhibit stability under accelerated stability testing conditions.
b. Resistance of retinal to oxidation when complexed with Hydroxysomes^{®} Calcium Phosphate Particulate Compositions and various antixoxidant components was evaluated using a proprietary assay. The results demonstrate that complexes having antixodant components as described above exhibit stability under accelerated stability testing conditions.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

Accordingly, the preceding merely illustrates the principles of the invention. It will be appreciated that those skilled in the art will be able to devise various arrangements which, although not explicitly described or shown herein, embody the principles of the invention and are included within the scope of the appended claims. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present invention, therefore, is not intended to be limited to the exemplary embodiments shown and described herein. Rather, the scope of the present invention is embodied by the appended claims.

## Claims

1. A shelf-life stable fine dry particulate retinal composition that includes a retinaldehyde present inside of the pores of nanoporous calcium particles and/or on the surface of the particles and the particles have diameters that range from 0.01 to 100 µm.

2. The fine dry particulate retinal composition according to Claim 1, wherein the weight percentage of retinaldehyde in the composition ranges from 1 to 50.

3. The fine dry particulate retinal composition according to Claims 1 or 2, wherein the nanoporous calcium particles are nanoporous calcium phosphate particles.

4. The fine dry particulate retinal composition according to Claim 3, wherein the nanoporous calcium phosphate particles are ceramic, uniform, rigid, spherical, nanoporous calcium phosphate particles.

5. The fine dry particulate retinal composition according to claim 4, wherein the uniform, rigid, spherical, nanoporous calcium phosphate particles have a diameter ranging from 1 to 10 µm.

6. The fine dry particulate retinal composition according to Claim 5, wherein the arithmetic mean diameter of all the particles of the uniform, rigid, spherical, nanoporous calcium phosphate particles is 2 µm or less.

7. The fine dry particulate retinal composition according to any one of claims 3 to 6, wherein the nanoporous calcium phosphate particles comprise pores ranging in size from 2 nm to 100 nm.

8. The fine dry particulate retinal composition according to any one of the preceding claims, wherein the composition further comprises an antioxidant component.

9. The fine dry particulate retinal composition according to Claim 8, wherein the antioxidant component comprises one or more of: BHT, Ascorbyl Palmitate, Thioctic Acid (Alpha Lipoic Acid), Calcium Ascorbate, a tocopherol, a mixed tocopherol composition and Resveratrol.

10. The fine dry particulate retinal composition according to Claim 9, wherein the antioxidant component is selected from the group consisting of: BHT and Ascorbyl Palmitate, Thioctic Acid (Alpha Lipoic Acid) and Calcium Ascorbate, Mixed Tocopherols and Thioctic Acid (Alpha Lipoic Acid) and Resveratrol, and Mixed Tocopherols and Thioctic Acid (Alpha Lipoic Acid).

11. A topical formulation comprising:
the fine dry particulate retinal composition according to any of Claims 1 to 10; and
a topical delivery vehicle.

12. A topical formulation according to Claim 11, wherein the topical delivery vehicle is an aqueous topical delivery vehicle.

13. A topical formulation according to claim 11 or claim 12, wherein the topical delivery vehicle is a cream.

## Patentansprüche

1. Während der Lagerfähigkeit haltbare, feine, trockene, teilchenförmige Retinalzusammensetzung, die ein Retinaldehyd umfasst, das innerhalb der Poren von nanoporösen Calciumteilchen und/oder an der Oberfläche der Teilchen vorliegt und wobei die Teilchen Durchmesser aufweisen, die im Bereich von 0,01 bis 100 µm liegen.

2. Feine, trockene, teilchenförmige Retinalzusammensetzung nach Anspruch 1, wobei der Gewichtsprozentsatz von Retinaldehyd in der Zusammensetzung im Bereich von 1 bis 50 liegt.

3. Feine, trockene, teilchenförmige Retinalzusammensetzung nach den Ansprüchen 1 oder 2, wobei die nanoporösen Calciumteilchen nanoporöse Calciumphosphatteilchen sind.

4. Feine, trockene, teilchenförmige Retinalzusammensetzung nach Anspruch 3, wobei die nanoporösen Calciumphosphatteilchen keramische, gleichförmige, steife, kugelförmige, nanoporöse Calciumphosphatteilchen sind.

5. Feine, trockene, teilchenförmige Retinalzusammensetzung nach Anspruch 4, wobei die gleichförmigen, steifen, kugelförmigen nanoporösen Calciumphosphatteilchen einen Durchmesser im Bereich von 1 bis 10 µm aufweisen.

6. Feine, trockene, teilchenförmige Retinalzusammensetzung nach Anspruch 5, wobei der arithmetische Mitteldurchmesser aller der Teilchen der gleichförmigen, steifen, kugelförmigen, nanoporösen Calciumphosphatteilchen 2 µm oder weniger beträgt.

7. Feine, trockene, teilchenförmige Retinalzusammensetzung nach einem der Ansprüche 3 bis 6, wobei die nanoporösen Calciumphosphatteilchen Poren umfassen, die größenmäßig im Bereich von 2 nm bis 100 nm liegen.

8. Feine, trockene, teilchenförmige Retinalzusammensetzung nach einem der vorhergehenden Ansprüche, wobei die Zusammensetzung ferner eine Antioxidationsmittelkomponente umfasst.

9. Feine, trockene, teilchenförmige Retinalzusammensetzung nach Anspruch 8, wobei die Antioxidationsmittelkomponente eines oder mehrere umfasst von: BHT, Ascorbylpalmitat, Thioctsäure (Alpha-Liponsäure), Calciumascorbat, einem Tocopherol, einer gemischten Tocopherolzusammensetzung und Resveratrol.

10. Feine, trockene, teilchenförmige Retinalzusammensetzung nach Anspruch 9, wobei die Antioxidationsmittelkomponente aus der Gruppe ausgewählt ist bestehend aus: BHT und Ascorbylpalmitat, Thioctsäure (Alpha-Liponsäure) und Calciumascorbat, gemischten Tocopherolen und Thioctsäure (Alpha-Liponsäure) und Resveratrol und gemischten Tocopherolen und Thioctsäure (Alpha-Liponsäure).

11. Topische Formulierung umfassend:
die feine, trockene, teilchenförmige Retinalzusammensetzung nach einem der Ansprüche 1 bis 10; und
ein topisches Abgabevehikel.

12. Topische Formulierung nach Anspruch 11, wobei das topische Abgabevehikel ein wässriges topisches Abgabevehikel ist.

13. Topische Formulierung nach Anspruch 11 oder Anspruch 12, wobei das topische Abgabevehikel eine Creme ist.

## Revendications

1. Composition particulaire fine et sèche de rétinal à durée de conservation stable qui inclut un rétinaldéhyde présent à l'intérieur des pores des particules de calcium nanoporeuses et/ou sur la surface des particules et les particules ont des diamètres qui se trouvent dans la plage de 0,01 à 100 µm.

2. Composition particulaire fine et sèche de rétinal selon la revendication 1, dans laquelle le pourcentage en poids de rétinaldéhyde dans la composition se trouve dans la plage de 1 à 50.

3. Composition particulaire fine et sèche de rétinal selon les revendications 1 ou 2, dans laquelle les particules de calcium nanoporeuses sont des particules de phosphate de calcium nanoporeuses.

4. Composition particulaire fine et sèche de rétinal selon la revendication 3, dans laquelle les particules de phosphate de calcium nanoporeuses sont des particules de phosphate de calcium nanoporeuses, sphériques, rigides, uniformes, céramiques.

5. Composition particulaire fine et sèche de rétinal selon la revendication 4, dans laquelle les particules de phosphate de calcium nanoporeuses, sphériques, rigides, uniformes ont un diamètre dans la plage de 1 à 10 µm.

6. Composition particulaire fine et sèche de rétinal selon la revendication 5, dans laquelle le diamètre moyen arithmétique de toutes les particules des particules de phosphate de calcium nanoporeuses, sphériques, rigides, uniformes est de 2 µm ou moins.

7. Composition particulaire fine et sèche de rétinal selon l'une quelconque des revendications 3 à 6, dans laquelle les particules de phosphate de calcium nanoporeuses comprennent des pores dans la plage de taille de 2 nm à 100 nm.

8. Composition particulaire fine et sèche de rétinal selon l'une quelconque des revendications précédentes, dans laquelle la composition comprend en outre un composant antioxydant.

9. Composition particulaire fine et sèche de rétinal selon la revendication 8, dans laquelle le composant antioxydant comprend un ou plusieurs de : BHT, palmitate d'ascorbyle, acide thioctique (acide alpha lipoïque), ascorbate de calcium, un tocophérol, une composition de tocophérols mixtes et resvératrol.

10. Composition particulaire fine et sèche de rétinal selon la revendication 9, dans laquelle le composant antioxydant est sélectionné dans le groupe constitué : de BHT et de palmitate d'ascorbyle, d'acide thioctique (acide alpha lipoique) et d'ascorbate de calcium, de tocophérols mixtes et d'acide thioctique (acide alpha lipoique) et de resvératrol, et de tocophérols mixtes et d'acide thioctique (acide alpha lipoïque).

11. Formulation topique comprenant :
la composition particulaire fine et sèche de rétinal selon l'une quelconque des revendications 1 à 10 ; et
un véhicule d'administration topique.

12. Formulation topique selon la revendication 11, dans laquelle le véhicule d'administration topique est un véhicule aqueux d'administration topique.

13. Formulation topique selon la revendication 11 ou la revendication 12, dans laquelle le véhicule d'administration topique est une crème.
